(19) Europäisches Patentamt
European Patent Office
Office européen des brevets

(11) Veröffentlichungsnummer : **0 044 420**
**B1**

(12) **EUROPÄISCHE PATENTSCHRIFT**

(45) Veröffentlichungstag der Patentschrift : **28.11.84**

(51) Int. Cl.³ : **C 07 D249/12**

(21) Anmeldenummer : **81104773.7**

(22) Anmeldetag : **22.06.81**

(54) **Hydroxyalkyl-1,2,4-triazolidin-3,5-dione und Verfahren zu ihrer Herstellung.**

(30) Priorität : **21.07.80 DE 3027551**

(43) Veröffentlichungstag der Anmeldung : **27.01.82 Patentblatt 82/04**

(45) Bekanntmachung des Hinweises auf die Patenterteilung : **28.11.84 Patentblatt 84/48**

(84) Benannte Vertragsstaaten : **AT BE DE FR GB IT SE**

(56) Entgegenhaltungen :
**DE-A- 2 554 866**
**US-A- 3 621 099**
**CHEMICAL ABSTRACTS, Vol. 63, No. 6, 13. September 1965, Columbus, Ohio, USA, G. PALAZZO "Thermal decomposition of N,N-dibenzylcarbamoyl azide", abstract no. 6999b**
**CHEMICAL ABSTRACTS, vol. 66, no. 22, 29. Mai 1967, Columbus, Ohio, USA, EASTMAN KODAK CO. "Photodevelopable, direct-recording silver halide emulsions", abstract no. 100194x**
**CHEMICAL ABSTRACTS, vol. 61, no. 3, 3. August 1964, Columbus, Ohio, USA, J. BOURDAIS "Heterocyclic series II., New syntheses and physiochemical properties of the 1,2,4-triazolidine-3,5-diones", abstract no. 3094h**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Giesecke, Henning, Dr.**
**Kalk-Muelheimer Strasse 400**
**D-5000 Koeln 80 (DE)**
Erfinder : **Merten, Rudolf, Dr.**
**Berta-von-Suttner-Strasse 55**
**D-5090 Leverkusen (DE)**
Erfinder : **Rottmaier, Ludwig**
**Bergstrasse 85**
**D-5068 Odenthal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 044 420 B1

**Beschreibung**

Gegenstand der vorliegenden Erfindung sind neue Hydroxy-alkyl-1,2,4-triazolidin-3,5-dione der allgemeinen Formel I

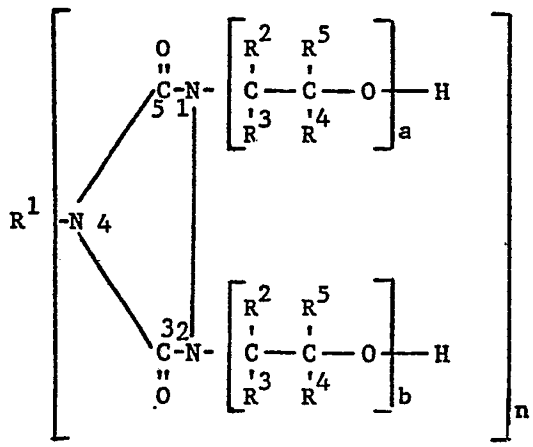

(I)

worin bedeuten

$R^1$

a) $C_nH_{2n+1}-$ $\quad$ n = 1 bis 18

b) $-(CH_2)_m-$ $\quad$ m = 2 bis 12

c) $-[CH_2-\underset{R^6}{CH}-O]_p-CH_2-\underset{R^6}{CH}-$ $\quad$ $R^6$ = H, $CH_3$; p = 1-9

d) $-[CH_2(CH_2)_q-\underset{R^7}{N}-]_r-(CH_2)_q-CH_2-$ $\quad$ $R^7$ = $C_1$-$C_4$-Alkyl; q = 1-2; r = 1-4

e) 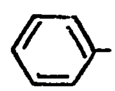 , 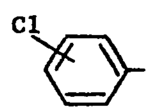

f) 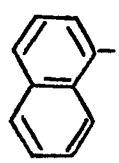 ,

g) 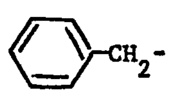

h) 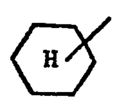

i) 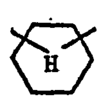

j) 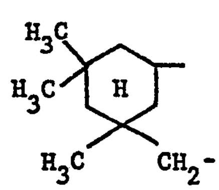

k) 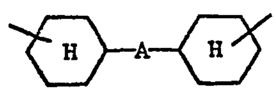

A = Alkylen mit 1-4 C-Atomen, O, $-N(CH_3)-$ ;

l) 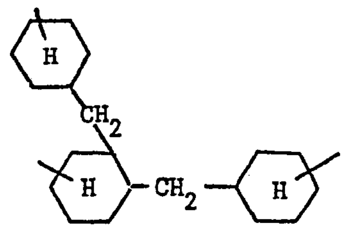

m) 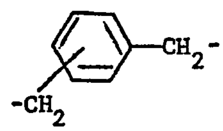

n) 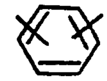

o) 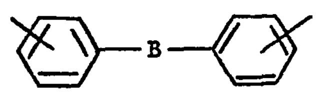

B = Alkylen mit 1-4 C-Atomen, O, $-N(CH_3)-$ ;

p) 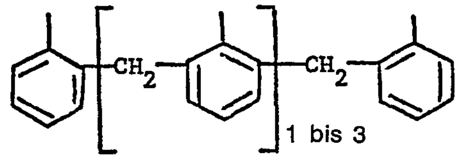

q) $C_4H_9-O-CH_2-CH_2-CH_2-$

r) $-(CH_2)_3-O-[CH_2]_{2\ \text{bis}\ 4}-O-(CH_2)_3-$

s) 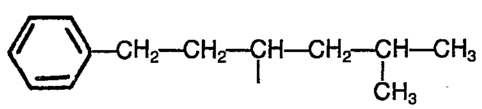

$R^2$, $R^3$, $R^4$, $R^5$ gleich oder verschieden, Wasserstoff einen aliphatischen $C_1$-$C_{10}$-, einen durch Chlor oder Brom substituierten $C_1$-$C_{10}$-aliphatischen, einen cycloaliphatischen $C_4$-$C_8$- oder araliphatischen $C_7$-$C_{17}$-Rest, einen aromatischen $C_6$-$C_{16}$- oder einen durch Chlor, Brom, $C_1$-$C_4$-Alkyl oder $C_1$-$C_4$-Alkoxy substituierten aromatischen $C_6$-$C_{16}$-Rest,

a und b gleich oder verschieden, eine Zahl von 1 bis 30 und

n eine Zahl von 1 bis 5, vorzugsweise 1 bis 3.

Vorzugsweise stellen $R^2$, $R^3$ und $R^4$ Wasserstoff und $R^5$ Wasserstoff, Methyl, Ethyl oder Phenyl, insbesondere $R^2$, $R^3$ und $R^4$ Wasserstoff sowie $R^5$ Wasserstoff und Methyl dar ; ferner a und b je eine Zahl von 1 bis 12, insbesondere die Zahl 1.

Die für die Herstellung der erfindungsgemäßen Hydroxyalkyl-1,2,4-triazolidin-3,5-dione der Formel I notwendigen Ausgangs-triazolidin-3,5-dione können nach verschiedenen Verfahren erhalten werden.

So können Amine der Formel II

$$R^1(NH_2)_n \qquad (II)$$

worin $R^1$ und n die für Formel I angegebene Bedeutung besitzen, mit Hydrazodicarbonamid (Verfahren 1) oder mit 1,2,4-Triazolidin-3,5-dion (Verfahren 2) bei 150-280 °C in Gegenwart oder Abwesenheit eines Lösungsmittels wie N-Methylpyrrolidon oder Lösungsmittelgemisches bei Drücken von 50 nbar bis 5 bar, gegenbenenfalls in Gegenwart eines sauren oder basischen Katalysators wie Alkoholate oder tert. Amine, unter Abspaltung von Ammoniak zu den Ausgangs-1,2,4-triazolidin-3,5-dionen umgesetzt werden.

Eine weitere Möglichkeit für die Herstellung der Ausgangs-1,2,4-triazolidin-3,5-dione besteht darin, daß man N-monosubstituierte Hydrazodicarbonamide der Formel III

$$[H_2N—CO—NH—NH—CO—NH—]_nR^1 \quad (III)$$

worin $R^1$ und n die in Formel I angegebene Bedeutung haben, unter den Bedingungen, wie vorstehend für die Verfahren 1 und 2 angegeben, unter Ammoniakabspaltung zu den Ausgangs-1,2,4-triazolidin-3,5-dionen umsetzt. Die N-monosubstituierten Hydrazodicarbonamide der Formel III können nach bekannten Verfahren aus Semicarbazid und Isocyanaten der Formel IV

$$R^1(NCO)_n \quad (IV)$$

worin $R^1$ und n die für Formel I angegebene Bedeutung haben, erhalten werden.

Die erfindungsgemäßen Hydroxyalkyl-1,2,4-triazolidin-3,5-dione der allgemeinen Formel I werden erhalten, indem man 1,2,4-Triazolidin-3,5-dione der Formel V

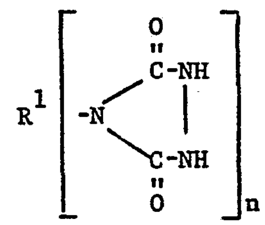

(V)

worin $R^1$ und n die in Formel I angegebene Bedeutung haben, mit Alkylenoxiden, insbesondere Ethylenoxid, Propylenoxid und Styroloxid, gegebenenfalls in Gegenwart eines geeigneten Katalysators, umsetzt.

Die Addition des Alkylenoxids an die NH-Gruppen der 1,2,4-Triazolidin-3,5-dione der Formel V kann sowohl bei Anwesenheit von sauren als auch alkalischen Katalysatoren vorgenommen werden. Vorzugsweise verwendet man jedoch bei der Herstellung der Polyole der Formel I basische Katalysatoren wie Tetraethylammoniumchlorid, tertiäre Amine wie Triethylamin und Dimethylanilin und Alkali- oder Erdalkalihydroxide oder deren Carbonate wie Calciumhydroxid oder Kaliumcarbonat. Es können jedoch auch Alkalihalogenide wie Lithiumchlorid eingesetzt werden. Die Menge an Katalysator liegt zwischen 0,05 und 3 %, bezogen auf die Reaktanten.

Eine bevorzugte Ausführungsform ist die Addition von einem Mol Alkylenoxid pro NH-Gruppe ohne Verwendung eines Katalysators.

Die Herstellung der Polyhydroxyverbindungen gemäß Formel I erfolgt vorzugsweise unter Einsatz äquivalenter Mengen, d. h. pro Mol 1,2,4-Triazolidin-3,5-dion der Formel V werden $n(a + b)$ Mole Alkylenoxid eingesetzt.

Die Reaktion der 1,2,4-Triazolidin-3,5-dione der Formel V mit dem Alkylenoxid wird vorzugsweise in inerten organischen Lösungsmitteln durchgeführt. Insbesondere geeignet sind polare organische Lösungsmittel wie dimethylsulfoxid, Tetramethylensulfon, Dimethylformamid, Dimethylacetamid und N-Methylpyrrolidon-2. Sehr gut geeignet als Lösungsmittel ist noch Wasser, wobei überraschenderweise keine Reaktion des Ethylenoxids mit dem Wasser zu beobachten ist. Dabei ist es nicht unbedingt erforderlich, die 1,2,4-Triazolidin-3,5-dione der Formel V, gelöst mit dem Alkylenoxid, in Reaktion zu bringen. Die Reaktion kann auch in Suspension der 1,2,4-Triazolidin-3,5-dione der Formel V erfolgen, wobei das gebildete Polyol dann in Lösung geht, so daß das Ende der Reaktion durch eine Klare Lösung angezeigt wird. Auch können in die entstehende Polyollösung 1,2,4-Triazolidin-3,5-dion der Formel V und Alkylenoxid gemeinsam zugesetzt werden, so daß die Lösungsmittelmenge sehr niedrig gehalten wird. Die Menge an Lösungsmittel sollte aus wirtschaftlichen Gründen sehr niedrig sein und kann zwischen 0,3 Gew.-Teilen und 20 Gew.-Teilen Lösungsmittel, bezogen auf ein Gew-Teil Reaktanten liegen. Nach beendeter Reaktion kann das Lösungsmittel durch Anlegen von Vakuum entfernt und der verbleibende viskose Rückstand durch übliche Arbeitsmethoden wie Umkristallisieren gereinigt werden. Häufig kann jedoch auf eine Reinigung verzichtet und das Rohprodukt sofort weiterverarbeitet werden.

Die Reaktion wird vorzugsweise bei Temperaturen von 25 °C bis 200 °C, besonders bevorzugt bei 80 °C bis 150 °C durchgeführt.

Die Reaktionszeiten liegen im allgemeinen zwischen 30 Minuten und mehreren Tagen, können in besonderen Fällen jedoch auch darüber oder darunter liegen. Durch entsprechende Wahl der Reaktionsbedingungen, z. B. Druck, werden kürzere Reaktionszeiten erreicht.

Eine besonders bevorzugte Ausführungsform ist die Umsetzung von 1,2,4-Triazolidin-3,5-dionen der Formel V in einem niederen Dialkylformamid (z. B. Dimethylformamid) mit Ethylenoxid bei einer Temperatur von 120 °C.

Das Lösungsmittel wird nach der Reaktion im Wasserstrahlvakuum entfernt und dann bei 0,2 mbar und 90-100 °C bis zur Gewichtskonstanz eingeengt. Der entstehende viskose Rückstand kristallisiert

häufig sehr schnell durch und kann nach Aufschlämmen in Methanol, Ethanol, Isopropanol, Aceton oder Gemischen davon abgesaugt werden. Die erhaltenen, nahezu reinen Poly-2-hydroxy-ethyl-1,2,4-triazolidin-3,5-dione der Formel I können durch Umkristallisation in den oben zitierten Lösungen noch weiter gereinigt werden.

Die erfindungsgemäß hergestellten Hydroxyalkyl-1,2,4-triazolidin-3,5-dione können als Vernetzer in hydroxylgruppenhaltige bekannte Polyester aus z. B. Adipinsäure, Phthalsäure, Diglykol eingebaut werden und werden zur Herstellung von Hart- oder Weichschaum- Polyurethanen verwendet.

Die erfindungsgemäßen Verbindungen der Formel I, insbesondere wenn a und b = 1 sind, können als Starter zur Herstellung von hydroxylgruppenhaltigen Polyethern und die wiederum, je nach Molgewicht, zur Herstellung von Hart- oder Weichschaum-Polyurethanen verwendet werden.

Die in den Beispielen angegebenen Teile und Prozente beziehen sich auf das Gewicht, sofern nicht anders vermerkt.

## Beispiele

Herstellung der Ausgangs-1,2,4-triazolidin-3,5-dione der Formel V.

### Ausgangsstoff 1

Zu einer Lösung von 111,5 g Semicarbazidhydrochlorid in 700 g Wasser wird so lange in kleinen Portionen Soda zugesetzt, bis keine Gasentwicklung mehr zu beobachten ist. Dann wird bei 40 °C eine lösung aus 119 g Phenylisocyanat in 100 g Aceton zugetropft. Zur Vervollständigung der Reaktion wird 2 Stunden bei 40 °C nachgerührt und der gebildete Niederschlag durch Absaugen isoliert.

Der über Nacht an der Luft getrocknete Niederschlag wird in 300 g Sulfolan suspendiert und auf 205 °C aufgeheizt, wobei ab 160 °C durch Anlegen eines Wasserstrahlvakuums von 420 mbar das freiwerdende Ammoniak abgezogen wird. Nach einer Reaktionszeit von 5 Stunden wird bei einem Druck von 0,3 mbar das Lösungsmittel größtenteils entfernt und der verbliebene Rückstand aus n-Butanol umkristallisiert. Nach dem Absaugen und Trocknen werden 134 g 4-Phenyl-1,2,4-triazolidin-3,5-dion vom Fp. = 202-203 °C (Lit. 203 °C) erhalten.

### Ausgangsstoff 2

600 g Hydrazodicarbonamid und 150 g Ethylendiamin werden in 500 ml N-Methylpyrrolidon 4 Stunden bei 175 °C und 20 Stunden bei 200 °C gerührt. Beim Abkühlen fällt ein Niederschlag aus, der abgesaugt und mit Ethanol gewaschen wird. Man erhält 462 g (80 % d. Th.) 1,2-Ethandiyl-4,4'-bis-1,2,4-triazolidin-3,5-dion als farblose Kristalle vom Fp. 330 °C.

$C_6H_6N_6O_4$ (228,2)

| | | | |
|---|---|---|---|
| ber. | C 31,58 % | H 3,53 % | N 36,83 % |
| gef. | C 31,4 % | H 3,6 % | N 36,8 % |

IR (KBr) : 1 731, 1 673 $cm^{-1}$ (C=O).

### Ausgangsstoff 3

360 g Hydrazodicarbonamid und 807 g Stearylamin und 1 ml Zinn(II)-dioctoat werden in 1 l N-Methylpyrrolidon 4 Stunden bei 175 °C und 6 Stunden bei 200 °C gerührt. Beim Abkühlen fällt ein Niederschlag aus, der abgesaugt und mit Wasser gewaschen wird. Man erhält 955 g (90 % d. Th.) 4-Stearyl-1,2,4-triazolidin-3,5-dion als farblose Kristalle vom Fp. 118-120 °C.

$C_{20}H_{39}N_3O_2$ (353,6)

| | | | |
|---|---|---|---|
| ber. | C 67,94 % | H 11,12 % | N 11,89 % |
| gef. | C 67,9 % | H 11,1 % | N 11,9 % |

MS (m/e) : Molpeak 353.

### Ausgangsstoff 4

111,5 g Semicarbazidhydrochlorid werden in 250 ml $H_2O$ gelöst und mit Soda neutralisiert. Anschließend werden bei Raumtemperatur unter Kühlung und starkem Rühren 169 g 1-Naphthylisocyanat in 100 ml Dioxan zugetropft. Die Mischung wird 1 Stunde bei Raumtemperatur nachgerührt. Man saugt vom Niederschlag ab und wäscht ihn mit $H_2O$.

Der feuchte Niederschlag wird in 1 l Sulfolan suspendiert und so lange bei 150 °C gerührt, bis kein $H_2O$ mehr abdestilliert. Anschließend wird die Suspension 3 Stunden bei 210 °C und 30 mbar gerührt. Beim Abkühlen entsteht ein Niederschlag, der abgesaugt und verworfen wird. Die Mutterlauge wird im

Hochvakuum eingeengt und der Rückstand mit 300 ml 10 %iger Natronlauge extrahiert. Beim Ansäuern der alkalischen Lösung mit Salzsäure entsteht ein Niederschlag, der abgesaugt und mit Wasser gewaschen wird.

Man erhält 123 g (54 % d. Th.) 4-Naphthyl-1,2,4-triazolidin-3,5-dion als farblose Kristalle vom Fp. 283-286 °C.

$C_{12}H_9N_3O_2$ (227,2)

| | | | |
|---|---|---|---|
| ber. | C 63,43 % | H 3,99 % | N 18,50 % |
| gef. | C 63,5 % | H 3,9 % | N 18,7 % |

$^1H$-NMR ($d_7$-DMF) = 7,4-8,2 (m, 7H arom.) 10,43 ppm (s, 2H)

Ausgangsstoff 5

60 g Hydrazodicarbonamid und 29 g 1,6-Diaminohexan werden in 100 ml Sulfolan 2 Stunden bei 175 °C und 9 Stunden bei 200 °C gerührt. Beim Abkühlen fällt ein Niederschlag aus, der abgesaugt und aus Wasser umkristallisiert wird. Man erhält 36 g (51 % d. Th.) 1,6-Hexandiyl-4,4'-bis-1,2,4-triazolidin-3,5-dion als farblose Kristalle vom Fp. : 215-217 °C.

$C_{10}H_{16}N_6O_4$ (284,3)

| | | | |
|---|---|---|---|
| ber. | C 42,25 % | H 5,67 % | N 29,51 % |
| gef. | C 42,5 % | H 5,7 % | N 29,2 % |

Ausgangsstoff 6

111,5 g Semicarbazidhydrochlorid werden in 400 ml $H_2O$ gelöst und mit Soda neutralisiert. Anschließend werden unter starkem Rühren bei Raumtemperatur 57 g Methylisocyanat, gelöst in 300 ml Dioxan, innerhalb von 1 Stunde zugetropft. Man rührt 2 Stunden bei Raumtemperatur nach und saugt vom entstandenen Niederschlag ab. Der Niederschlag wird in 1 l N-Methylpyrrolidon suspendiert und 10 Stunden bei 200 °C und 300 mbar pyrrolysiert. Anschließend wird das Lösungsmittel im Vakuum abdestilliert und der Rückstand aus Ethanol umkristallisiert. Man erhält 96 g (83 % d. Th.) 4-Methyl-1,2,4-triazolidin-3,5-dion als farblose Kristalle vom Fp. : 230-232 °C (Lit. 232-233 °C).

Ausgangsstoff 7

102 g 1,4-Butandiolbis-(3-aminopropylether) und 120 g Hydrazodicarbonamid werden in 300 ml N-Methylpyrrolidon 1 Stunde bei 150 °C, 2 Stunden bei 175 °C und 5 Stunden bei 200 °C gerührt. Beim Abkühlen fällt ein Niederschlag aus, der abgesaugt und mit 200 ml Acetonitril ausgekocht wird. Als Rückstand erhält man 142 g (76 % d. Th.) 1,4-Butandiol-bis-[3-(3,5-dioxo-1,2,4-triazolidin-4-yl)-propyl-ether] als farblose Kristalle vom Fp. : 152-154 °C.

$C_{14}H_{24}N_6O_6$ (372,4)

| | | | |
|---|---|---|---|
| ber. | C 45,15 % | H 6,50 % | N 22,57 % |
| gef. | C 45,2 % | H 6,6 % | N 22,6 % |

IR (KBr) : 1 768, 1 674 $cm^{-1}$ (C=O).

Ausgangsstoff 8

131 g 3-Butoxypropylamin und 120 g Hydrazodicarbonamid werden in 300 ml N-Methylpyrrolidon 1 Stunde bei 150 °C, 2 Stunden bei 175 °C und 6 Stunden bei 200 °C gerührt. Beim Abkühlen fällt ein Niederschlag aus, der abgesaugt und aus Cyclohexan umkristallisiert wird. Man erhält 152 g (75 % d. Th.) 4-(3-Butoxypropyl)-1,2,4-triazolidin-3,5-dion als farblose Kristalle vom Fp. 83-85 °C.

$C_9H_{17}N_3O_3$ (215,2)

| | | | |
|---|---|---|---|
| ber. | C 50,22 % | H 7,96 % | N 19,52 % |
| gef. | C 50,2 % | H 8,1 % | N 19,8 % |

$^1H$-NMR ($CDCl_3$) = 0,90 (t, 3H ; J = 6 Hz), 1,0-2,2 (m, 6H), 3,2-3,9 (m, 6H) 9,21 ppm (s, 2H).

Ausgangsstoff 9

191 g 3-Amino-5-methyl-1-phenylhexan und 120 g Hydrazocarbonamid werden in 250 ml N-Methylpyrrolidon 1 Stunde bei 150 °C, 1 Stunde bei 175 °C und 2 Stunden bei 200 °C gerührt. Beim Abbkühlen fällt ein Niederschlag aus, der abgesaugt und aus Cyclohexan umkristallisiert wird. Man erhält

252 g (92 % d. Th.) 4-(5-Methyl-1-phenylhexyl-3)-1,2,4-triazolidin-3,5-dion als farblose Kristalle vom Fp. 114-115 °C.

$C_{15}H_{21}N_3O_3$ (275,3)
ber. C 65,43 % H 7,96 % N 15,25 %
gef. C 65,2 % H 7,4 % N 15,4 %

Ir (KBr) : 1 767, 1 671 $cm^{-1}$ (C=O).

Ausgangsstoff 10

101 g (1,2,4-Triazolidin-3,5-dion) und 57 g 1,4-Diaminocyclohexan werden in 500 ml N-Methylpyrrolidon 4 h auf 175 °C und 30 Stunden auf 200 °C unter Rühren erhitzt. Beim Abkühlen kristallisiert ein Niederschlag aus, der abgesaugt, mit Ethanol gewaschen und getrocknet wird. Man erhält 110 g (78 % d. Th.) 1,4-Cyclohexandiyl-4,4'-bis-(1,2,4-triazolidin-3,5-dion) als farblose Kristalle vom Fp. > 300 °C.

$C_{10}H_{14}N_6O_4$ (282,2)
ber. C 42,56 % H 4,96 % N 29,75 %
gef. C 42,7 % H 5,1 % N 29,1 %

MS (m/e) : Molpeak 282

Beispiel 1

Zu einer Lösung aus 177 g 4-Phenyl-1,2,4-triazolidin-3,5-dion (Ausgangsstoff 1) und 2,5 g Tetraethylammoniumchlorid in 100 g Dimethylformamid werden in 6,5 Stunden bei 120 °C 88 g Ethylenoxid so eingeleitet, daß kein Ethylenoxid entweicht. Anschließend wird das Lösungsmittel im Vakuum abdestilliert und der viskose Rückstand in 250 ml Ethanol unter Erwärmen gelöst. Beim Abkühlen fällt ein Niederschlag aus, der abgesaugt und mit Ethanol gewaschen wird. Man erhält 199 g 1,2-Bis-(2-hydroxyethyl)-4-phenyl-1,2,4-triazolidin-3,5-dion als farblose Kristalle vom Fp. 133 °C.

$C_{12}H_{15}N_3O_4$ (265,3)
ber. C 54,33 % H 5,70 % N 15,81 %
gef. C 54,3 % H 5,6 % N 16,1 %

Beispiel 2

Zu einer Suspension von 228 g 1,2-Ethandiyl-4,4'-bis-1,2,4-triazolidin-3,5-dion (Ausgangsstoff 2) und 2 g Triethylamin in 1 kg Dimethylformamid werden bei 125 °C innerhalb von 6 Stunden 176 g Ethylenoxid so eingeleitet, daß kein Ethylenoxid entweicht. Durch Anlegen von Vakuum wird bei 40 mbar der größte Teil des Lösungsmittels entfernt. Der erhaltene Rückstand von 483 g wird in 500 ml eines Lösungsmittelgemisches aus 7 Vol.-Teilen Aceton und 3 Vol.-Teilein Isopropanol unter Erwärmen gelöst. Beim Abkühlen fällt ein Niederschlag aus, der abgesaugt und im Vakuum getrocknet wird.

Man erhält 330 g 1,2-Ethandiyl-4,4'-bis-[1,2-bis-(2-hydroxyethyl)-1,2,4-triazolidin-3,5-dion] als farblose Kristalle vom Fp. 153-155 °C.

$C_{14}H_{24}N_6O_8$ (404,4)
ber. C 41,58 % H 5,98 % N 20,78 %
gef. C 41,4 % H 5,7 % N 20,9 %

Beispiel 3

Zu einer Schmelze von 530 g 4-Stearyl-1,2,4-triazolidin-3,5-dion (Ausgangsstoff 3) und 3 g Triethylamin werden bei 120 °C innerhalb von 6 Stunden 132 g Ethylenoxid so eingeleitet, daß kein Ethylenoxid entweicht. Das Reaktionsgemisch wird abgekühlt und anschließend in 2,5 l Aceton gelöst. Beim Abkühlen fällt ein Niederschlag aus, der abgesaugt und mit Aceton gewaschen wird. Man erhält 575 g 1,2-Bis-(2-hydroxyethyl)-4-stearyl-1,2,4-triazolidin-3,5-dion als farblose Kristalle vom Fp. 68-79 °C.

$C_{24}H_{47}N_3O_4$ (441,7)
ber. C 65,27 % H 10,73 % N 9,52 %
gef. C 65,6 % H 10,7 % N 9,4 %

Beispiel 4

Zu 22,7 g 4-(1-Naphthyl)-1,2,4-triazolidin-3,5-dion (Ausgangsstoff 4) und 0,2 g Triethylamin in 100 g

Dimethylformamid werden bei 120 °C 8,8 g Ethylenoxid eingeleitet. Nach Beendigung der Reaktion wird das Lösungsmittel in Vakuum abdestilliert und der Rückstand in Isopropanol unter Erwärmen gelöst. Beim Abkühlen fällt ein Niederschlag aus, der abgesaugt und getrocknet wird. Man erhält 21 g 1,2-Bis-(2-hydroxyethyl)-4-(1-naphthyl)-1,2,4-triazolidin-3,5-dion vom Fp. 211-212 °C.

IR- und NMR-Spektren sowie die Elementaranalyse bestätigen die angenommene Struktur.

$C_{16}H_{17}N_3O_4$ (315,3)

| | | | |
|---|---|---|---|
| ber. | C 60,95 % | H 5,43 % | N 13,33 % |
| gef. | C 60,7 % | H 5,2 % | N 13,4 % |

Beispiel 5

Zu 142 g 1,6-Hexandiyl-4,4'-bis-1,2,4-triazolidin-3,5-dion (Ausgangsstoff 5) und 1 g Tetraethylammoniumchlorid in 250 g Dimethylformamid werden bei 120 °C innerhalb von 5 Stunden 88 g Ethylenoxid so eingeleitet, daß kein Ethylenoxid entweicht. Durch Anlegen von Vakuum wird bei 50 mbar der größte Teil des Lösungsmittels entfernt. Der erhaltene Rückstand von 235 g wird in einem Gemisch aus 300 ml Isopropanol und 500 ml Aceton unter Erwärmen gelöst. Beim Abkühlen fällt ein Niederschlag aus, der abgesaugt und im Vakuum getrocknet wird.

Man erhält 162 g 1,6-Hexandiyl-4,4'-bis-[1,2-bis-(2-hydroxyethyl)-1,2,4-triazolidin-3,5-dion] als farblose Kristalle vom Fp. = 128 °C.

IR- und NMR-Spektren sowie die Elementaranalyse bestätigen die angenommene Struktur.

$C_{18}H_{32}N_6O_8$ (460,5)

| | | | |
|---|---|---|---|
| ber. | C 46,94 % | H 7,00 % | N 18,25 % |
| gef. | C 47,2 % | H 6,8 % | N 18,2 % |

Beispiel 6

a) Zu 575 g 4-Methyl-1,2,4-triazolidin-3,5-dion (Ausgangsstoff 6) und 5 g Lithiumchlorid in 1 000 g Dimethylformamid werden bei 110 °C bis 120 °C innerhalb von 8 Stunden 440 g Ethylenoxid so eingeleitet, daß kein Ethylenoxid entweicht. Das Lösungsmittel wird durch Anlegen von Vakuum abdestilliert. Der Rückstand wird in 1 l Essigsäurethylester durch Erwärmen gelöst. Beim Abkühlen kristallisiert ein Niederschlag aus, der abgesaugt und getrocknet wird.

Man erhält 675 g 1,2-Bis-(2-hydroxyethyl)-4-methyl-1,2,4-triazolidin-3,5-dion vom Fp. = 126-127 °C (aus Ethanol).

IR- und NMR-Spektren sowie die Elementaranalyse bestätigen die angenommene Struktur.

$C_7H_{13}N_3O_4$ (203,2)

| | | | |
|---|---|---|---|
| ber. | C 41,37 % | H 6,45 % | N 20,68 % |
| gef. | C 41,5 % | H 6,2 % | N 20,6 % |

b) 291 g Terephthalsäuredimethylester, 203 g 1,2-Bis-(2-hydroxyethyl)-4-methyl-1,2,4-triazolidin-3,5-dion aus Beispiel 6 a), 31 g 1,2-Ethandiol und 92 g Glycerin werden aufgeschmolzen, mit 1 g Bleiacetat, 0,5 g Titantetrabutylat und 50 ml Xylol versetzt und innerhalb von 8 Stunden auf 200 °C aufgeheizt. Dann wird in 3 Stunden auf 220 °C erhitzt und durch Anlegen von Vakuum über 4 Stunden weiterkondensiert. Es werden 517 g eines braunen, bei Raumtemperatur, spröden Harzes enthalten. 30 g dieses Harzes werden in 70 g eines technischen m-Kresols gelöst, mit 0,9 g einer stabilisierten Titantetrabutylatlösung (hergestellt durch Erwärmen von 1 Gew.-Teil Titantetrabutylat und 2 Gew.-Teilen Kresol) versetzt, auf eine entfettete Glasplatte gestrichen und 20 Minuten bei 200 °C sowie 10 Minuten bei 300 °C eingebrannt. Es wird ein elastischer Film von glatter Oberfläche und hoher Erweichungstemperatur erhalten.

Beispiel 7

Zu 55,8 g 1,4-Butanbisoxypropyl-3-diyl-[1,2,4-triazolidin-3,5-dion-4-yl] (Ausgangsstoff 7) und 0,3 g Trimethyl-phenylammoniumchlorid in 100 g Dimethylformamid werden bei 110 °C 26,4 g Ethylenoxid eingeleitet. Nach Beendigung der Reaktion wird das Lösungsmittel durch Anlegen von Vakuum abdestilliert. Es verbleiben 85 g eines hellbraunen Öles, welches zur weiteren Reinigung in einem Gemisch aus 7 Vol.-Teilen Chloroform und 3 Vol.-Teilen Methanol gelöst und über Kieselgel filtriert wird. Nach dem Abdestillieren des Lösungsmittels, zuletzt bei 70 °C und 0,3 mbar werden 75 g eines gelblichen Öles erhalten, welches hauptsächlich aus 1,4-Butanbisoxypropyl-3-diyl-4,4'-bis-[1,2-bis-(2-hydroxyethyl)-1,2,4-triazolidin-3,5-dion] besteht.

IR- und NMR-Spektren sowie die Elementaranalyse bestätigen die angenommene Struktur

$C_{22}H_{40}N_6O_{10}$ (548,6)

| | | | | |
|---|---|---|---|---|
| ber. | C 48,16 % | H 7,35 % | N 15,32 % | OH 12,40 % |
| gef. | C 47,8 % | H 7,0 % | N 15,5 % | OH 12,35 % |

Beispiel 8

Analog Beispiel 7 werden 53,75 g 4-(3-Butoxypropyl)-1,2,4-triazolidin-3,5-dion (Ausgangsstoff 8) mit 22 g Ethylenoxid umgesetzt und aufgearbeitet. Es werden 69 g eines gelben Öles erhalten, welches hauptsächlich aus 1,2-Bis-(2-hydroxyethyl)-4-(3-butoxypropyl)-1,2,4-triazolidin-3,5-dion besteht.
IR- und NMR-Spektren sowie die Elementaranalyse bestätigen die angenommene Struktur.

$C_{13}H_{25}N_3O_5$ (303,4)

| | | | | |
|---|---|---|---|---|
| ber. | C 51,46 % | H 8,31 % | N 13,85 % | OH 11,21 % |
| gef. | C 51,2 % | H 8,1 % | N 14,1 % | OH 11,0 % |

Beispiel 9

Zu 82,5 g 4-(5-Methyl-2-phenylhexyl-3)-1,2,4-triazolidin-3,5-dion (Ausgangsstoff 9) und 0,3 g Trimethylbenzylammoniumhydroxid in 150 g Dimethylacetamid werden 26,4 g Ethylenoxid eingeleitet. Das Lösungsmittel wird in Vakuum abdestilliert, der Rückstand durch Erwärmen in 150 ml Essigsäureethylester gelöst, 10 g pulverisierte A-Kohle zugesetzt und heiß filtriert. Beim Abkühlen kristallisiert ein Niederschlag aus, der abgesaugt und getrocknet wird. Man erhält 68 g 1,2-Bis-(2-hydroxyethyl)-4-(5-methyl-1-phenylhexyl-3)-1,2,4-triazolidin-3,5-dion als farblose Kristalle vom Fp. = 114 °C.
IR- und NMR-Spektren sowie die Elementaranalyse bestätigen die angenommene Struktur.

$C_{19}H_{29}N_3O_4$ (363,4)

| | | | |
|---|---|---|---|
| ber. | C 62,79 % | H 8,04 % | N 11,56 % |
| gef. | C 62,7 % | H 7,9 % | N 11,4 % |

Beispiel 10

Zu 141 g 1,4-Cyclohexandiyl-bis-(1,2,4-triazolidin-3,5-dion-4-yl) (Ausgangsstoff 10) und 0,5 g Triethylamin in 250 g Dimethylformamid werden bei 120 °C 88 g Ethylenoxid eingeleitet. Das Lösungsmittel wird im Vakuum abdestilliert und der Rückstand durch Erwärmen in 600 ml Essigsäureethylester gelöst. Beim Abkühlen kristallisiert ein Niederschlag aus, der abgesaugt und getrocknet wird. Man erhält 174 g 1,4-Cyclohexandiyl-4,4'-bis-[1,2-bis-(2-hydroxyethyl)-1,2,4-triazolidin-3,5-dion] als farblose Kristalle vom Fp. = 263-265 °C.
IR- und NMR-Spektren sowie die Elementaranalyse bestätigen die angenommene Struktur.

$C_{18}H_{30}N_6O_8$ (458,5)

| | | | |
|---|---|---|---|
| ber. | C 47,15 % | H 6,60 % | N 18,33 % |
| gef. | C 47,0 % | H 6,7 % | N 18,3 % |

**Ansprüche**

1. Hydroxyalkyl-1,2,4-triazolidin-3,5-dione der allgemeinen Formel I

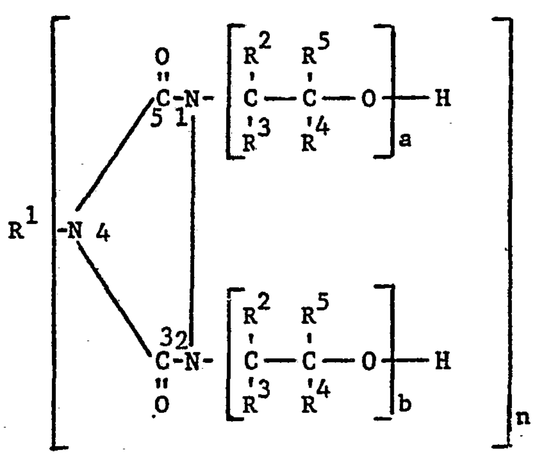

(I)

worin bedeuten :

$R^1$

a) $C_nH_{2n+1}$— n = 1 bis 18

b) —$(CH_2)_m$— m = 2 bis 12

c) 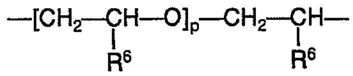

$R^6 = H, CH_3$
$p = 1\text{-}9$

d) 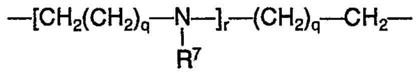

$R^7 = C_1\text{-}C_4$-Alkyl
$q = 1\text{-}2$
$r = 1\text{-}4$

e) 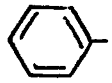 , 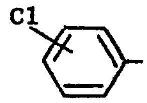

f) 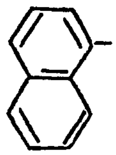 ,

g) 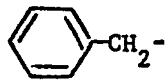

h) 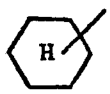

i) 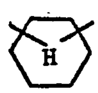

j) 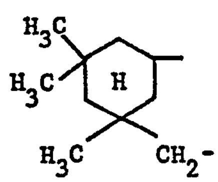

k) 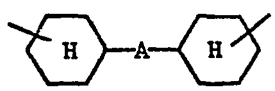

A = Alkylen mit 1-4 C-Atomen, O, $-N(CH_3)-$ ;

l) 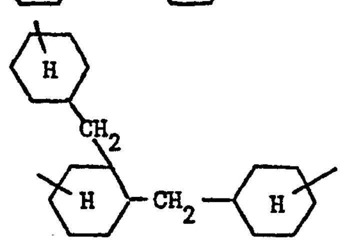

m) 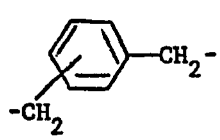

n) 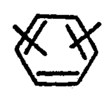

o) 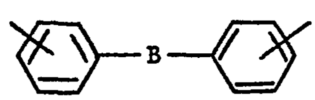

B = Alkylen mit 1-4 C-Atomen, O, $-N(CH_3)-$ ;

p) 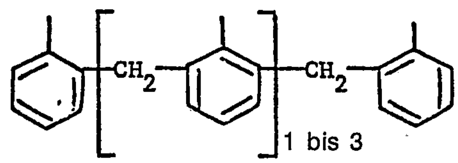

q) $C_4H_9—O—(CH_2)_3—$

r) $—(CH_2)_3—O—[CH_2]_{2\ bis\ 4}—O—(CH_2)_3—$

s) 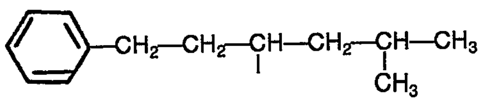

$R^2$, $R^3$, $R^4$, $R^5$ gleich oder verschieden, Wasserstoff, einen aliphatischen $C_1$-$C_{10}$-, einen durch Chlor oder Brom substituierten $C_1$-$C_{10}$-aliphatischen, einen cycloaliphatischen $C_4$-$C_8$- oder araliphatischen $C_7$-$C_{17}$-Rest, einen aromatischen $C_6$-$C_{16}$ oder einen durch Chlor oder Brom, $C_1$-$C_4$-Alkyl oder $C_1$-$C_4$-Alkoxi substituierten aromatischen $C_6$-$C_{16}$-Rest,

a und b gleich oder verschieden, eine Zahl von 1 bis 30, und

n eine Zahl von 1 bis 5.

2. Hydroxyalkyl-1,2,4-triazolidin-3,5-dione gemäß Anspruch 1, dadurch gekennzeichnet, daß $R^2$, $R^3$ und $R^4$ Wasserstoff sowie $R^5$ Wasserstoff, Methyl, Ethyl oder Phenyl bedeuten und a und b eine Zahl von 1-12 sowie n eine Zahl von 1 bis 3 darstellen.

3. Hydroxyalkyl-1,2,4-triazolidin-3,5-dione gemäß den Ansprüchen 1 und 2, dadurch gekennzeichnet, daß a und b die Zahl 1 darstellen.

4. Verfahren zur Herstellung von Hydroxyalkyl-1,2,4-triazolidin-3,5-dionen gemäß Anspruch 1, dadurch gekennzeichnet, daß 1,2,4-Triazolidin-3,5-dione der Formel

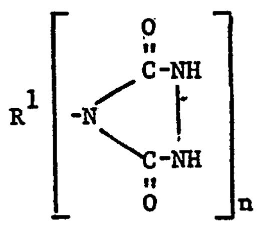

worin $R^1$ und n die in Anspruch 1 angegebene Bedeutung haben, mit mindestens einem Alkylenoxid der Formel

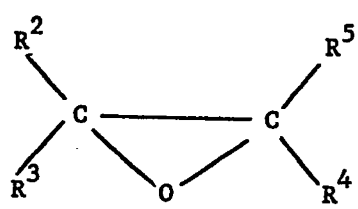

worin $R^2$, $R^3$, $R^4$ und $R^5$ die in Anspruch 1 angegebene Bedeutung besitzen, in Gegenwart oder Abwesenheit eines Lösungsmittels umgesetzt werden.

## Claims

1. Hydroxyalkyl-1,2,4-triazolidine-3,5-diones of the general formula I

(See formula I, page 12)

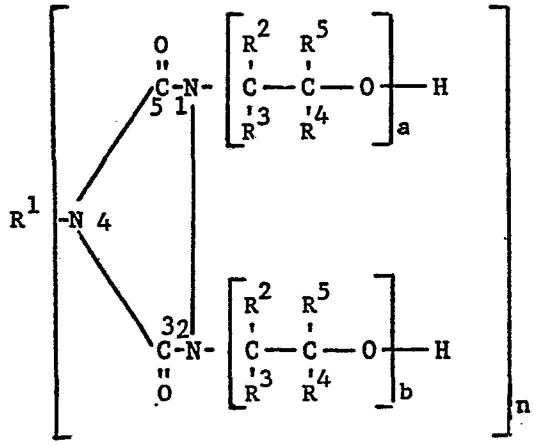 (I)

wherein

$R^1$ denotes

a) $C_nH_2n_{+1}$— $n = 1$ to 18

b) $—(CH_2)_m—$ $m = 2$ to 12

c) $—[CH_2—\underset{R^6}{CH}—O]_p—CH_2—\underset{R^6}{CH}—$ $R^6 = H, CH_3$; $p = 1\text{-}9$

d) $—[CH_2(CH_2)_q—\underset{R^7}{N}—]_r—(CH_2)_q—CH_2—$ $R^7 = C_1\text{-}C_4$-alkyl; $q = 1\text{-}2$; $r = 1\text{-}4$

e) 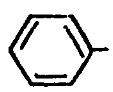 , 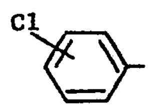

f) 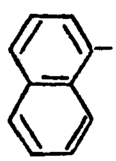 ,

g) 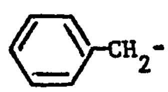

h) 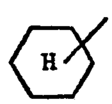

i) 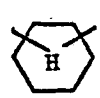

j) 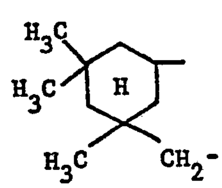

k) 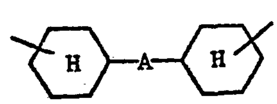 A = alkylen with 1-4 C-atoms, O, $—N(CH_3)—$ ;

l) 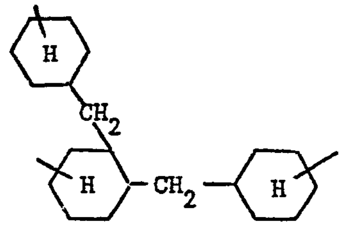

m) 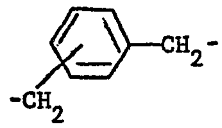

n) 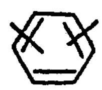

o) 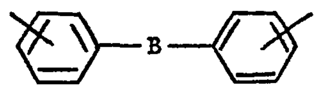 B = alkylene with 1-4 C-atoms, O, $-N(CH_3)-$;

p) 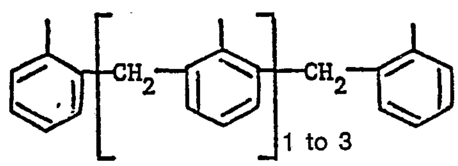

q) $C_4H_9-O-(CH_2)_3-$

r) $-(CH_2)_3-O-[CH_2]_{2\ to\ 4}-O-(CH_2)_3-$

s) 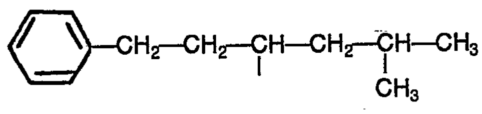

$R^2$, $R^3$, $R^4$ and $R^5$, which are identical or different, denote hydrogen, an aliphatic $C_1$-$C_{10}$-radical, a $C_1$-$C_{10}$-aliphatic radical substituted by chlorine or bromine, a cycloaliphatic $C_4$-$C_8$-radical or an araliphatic $C_7$-$C_{17}$-radical, an aromatic $C_6$-$C_{16}$-radical or an aromatic $C_6$-$C_{16}$-radical substituted by chlorine or bromine, $C_1$-$C_4$-alkyl or $C_1$-$C_4$-alkoxy,

a and b, which are identical or different, denote a number from 1 to 30, and

n denotes a number from 1 to 5.

2. Hydroxyalkyl-1,2,4-triazolidine-3,5-diones according to Claim 1, characterised in that $R^2$, $R^3$ and $R^4$ denote hydrogen and $R^5$ denotes hydrogen, methyl, ethyl or phenyl and a and b represent a number from 1-12 and n represents a number from 1 to 3.

3. Hydroxyalkyl-1,2,4-triazolidine-3,5-diones according to Claims 1 and 2, characterised in that a and b represent the number 1.

4. Process for the preparation of hydroxyalkyl-1,2,4-triazolidine-3,5-diones according to Claim 1, characterised in that 1,2,4-triazolidine-3,5-diones of the formula

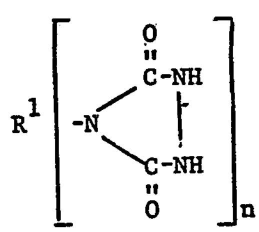

wherein $R^1$ and n have the meaning given in Claim 1, are reacted with at least one alkylene oxide of the formula

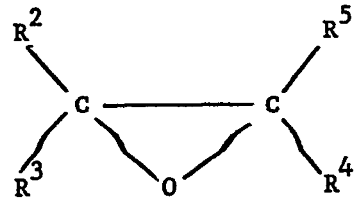

wherein $R^2$, $R^3$, $R^4$ and $R^5$ have the meaning given in Claim 1, in the presence or absence of a catalyst and in the presence or absence of a solvent.

**Revendications**

1. Hydroxyalkyl-1,2,4-triazolidine-3,5-diones de formule générale I

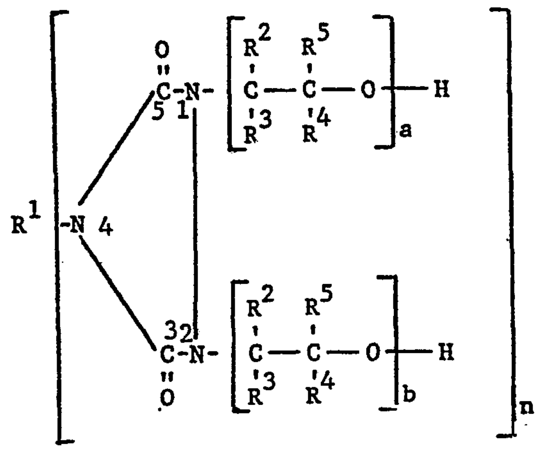

(I)

dans laquelle

$R^1$ représente

a) $C_nH_{2n+1}-$ — n valant 1 à 18

b) $-(CH_2)_m-$ — m valant 2 à 12

c) $-[CH_2-\underset{R^6}{CH}-O]_p-CH_2-\underset{R^6}{CH}-$ — $R^6$ représentant H, $CH_3$; p valant 1 à 9

d) $-[CH_2(CH_2)_q-\underset{R^7}{N}-]_r-(CH_2)_q-CH_2-$ — $R^7$ étant un groupe alkyle en $C_1$-$C_4$; q valant 1 ou 2; r valant 1 à 4

e) 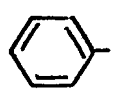 , 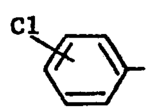

f) 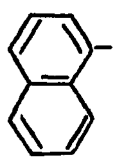 ,

g) 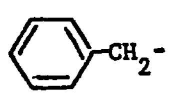

h) 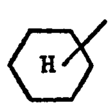

i) 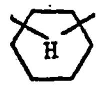

j) 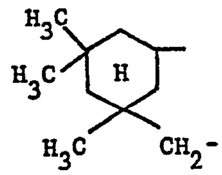

k) 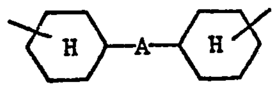

A représentant un groupe alkylène ayant 1 à 4 atomes de carbone, O, —N($CH_3$)— ;

l) 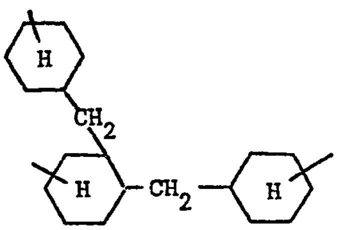

m) 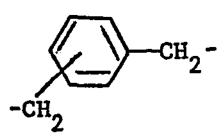

n) 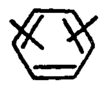

o) 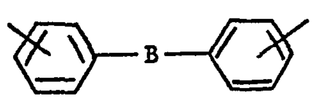

B représentant un groupe alkylène ayant 1 à 4 atomes de carbone, O, —N($CH_3$)— ;

p) 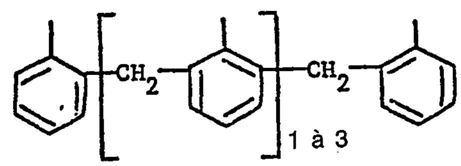

q) $C_4H_9—O—(CH_2)_3—$

r) $—(CH_2)_3—O—[CH_2]_{2\ à\ 4}—O—(CH_2)_3—$

s) 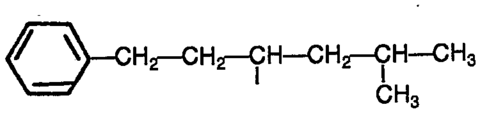

$R^2$, $R^3$, $R^4$ et $R^5$, identiques ou différents, représentent chacun un atome d'hydrogène, un radical aliphatique en $C_1$-$C_{10}$, un radical aliphatique en $C_1$-$C_{10}$ substitué par du chlore ou du brome, un radical cycloaliphatique en $C_4$ à $C_8$ ou araliphatique en $C_7$ à $C_{17}$, un radical aromatique en $C_6$ à $C_{16}$ ou un radical aromatique en $C_6$ à $C_{16}$ substitué par du chlore ou du brome, par un groupe alkyle en $C_1$-$C_4$ ou alcoxy en $C_1$ à $C_4$ ;

a et b, identiques ou différents, représentent chacun un nombre valant 1 à 30, et

n est un nombre valant 1 à 5.

2. Hydroxyalkyl-1,2,4-triazolidine-3,5-diones selon la revendication 1, caractérisées en ce que $R^2$, $R^3$ et $R^4$ représentent chacun un atome d'hydrogène et $R^5$ représente un atome d'hydrogène, un groupe

méthyle, éthyle ou phényle, a et b représentent chacun un nombre valant 1 à 12 et n est un nombre valant 1 à 3.

3. Hydroxyalkyl-1,2,4-triazolidine-3,5-diones selon les revendications 1 et 2, caractérisées en ce que a et b représentent chacun le nombre 1.

4. Procédé de préparation d'hydroxyalkyl-1,2,4-triazolidine-3,5-diones selon la revendication 1, caractérisé en ce qu'on fait réagir, en présence ou en l'absence d'un catalyseur et en présence ou en l'absence d'un solvant, des 1,2,4-triazolidine-3,5-diones de formule

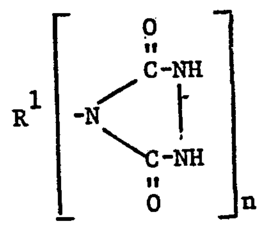

dans laquelle $R^1$ et n ont le sens indiqué à la revendication 1, avec au moins un oxyde d'alkylène de formule

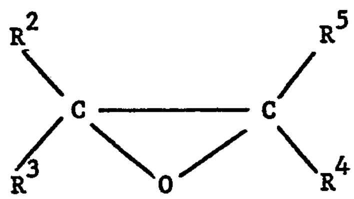

dans laquelle $R^2$, $R^3$, $R^4$ et $R^5$ ont le sens indiqué à la revendication 1.